# EUROPEAN PATENT SPECIFICATION

(11) **EP 0 481 020 B1**
(45) Date of publication and mention of the grant of the patent: **13.03.1996**
(21) Application number: 90917909.5
(22) Date of filing: 06.07.1990
(51) Int. Cl.: G01N 33/53, G01N 33/543, G01N 33/94, G01N 33/569, G01N 33/74

(54) **Silver enhanced gold-labelled immuno assay method**
Durch Silber intensiviertes, auf Goldmarkierung beruhendes Immuntestverfahren
Méthode d'immunoessai avec marquage à l'or intensifié à l'aide d'argent

(30) Priority: 06.07.1989 GB 8915512
(43) Date of publication of application: 22.04.1992
(73) Proprietor: SECRETARY OF STATE FOR HEALTH IN HER BRITANNIC MAJESTY'S GOV. OF THE UNITED KINGDOM OF GREAT BRITAIN AND NORTHERN IRELAND, London WC1B 5EP (GB)
(72) Inventor: ROCKS, Bernard, Francis, Roedean, Brighton BN2 5RD (GB); BAILEY, Michael, Philip, East Sussex BN9 9RY (GB); BERTRAM, Vanessa, M., R., London SE5 9LH (GB)
(74) Representative: Skelton, Stephen Richard
(86) International application number: GB9001046
(87) International publication number: WO9101003

(56) References cited:
- EP-A- 0 158 746
- EP-A- 0 293 947
- Histochemistry (1986) 85:209
- Histochemistry (1985) 83:545

## Description

The present invention relates to a method of assaying whole blood for analytes capable of undergoing specific binding reactions, such analytes being, for example, specific antibodies or antigens, said antigens being for example proteins, viruses, hormones, drugs, nucleic acids and polysaccharides.

The present invention further provides a method of determining, quantitatively or qualitatively, the presence and/or amount of specific analytes, particularly antibodies, in whole blood.

Assay for specific antibodies in physiological fluids, whether of IgG, IgM, IgD or IgA classes, is the principle method of detecting infection by antigens, particularly of bacterial or viral infections, to which the antibodies are specific. The use of enzyme linked immunosorbent assay (ELISA) methods for the detection of antibodies is well known.

Generally, in an ELISA method, the antibody or antigen being assayed is captured by a respective antigen or antibody immobilised on a substrate and is then exposed to a secondary antibody labelled with an enzyme. The secondary antibody binds to the captured antibody or antigen such that its presence or amount may be assayed as related to bound enzyme activity,. usually by an enzymic reaction producing a colour change.

Such methods are of particular importance at present due to the widespread screening necessitated by the discovery of human immunodeficiency virus (HIV), previously referred to as human T-cell lymphotropic virus type 3 (HTLV-III) or lymphadenopathy associated virus (LAV), and its role in aquired immune deficiency syndrome (AIDS).

Tests for detecting infection by HIV are of particular value in studying the spread of the epidemic, as an aid to the diagnosis of AIDS and in screening blood to avoid the use of donations from infected people. Since HIV may be transmitted by blood transfusion and by blood products (eg: Factor VIII as used for treatment of haemophilia) the testing of all blood donations has become a necessity. Screening tests currently used rely upon detection of specific antibodies in bodily fluids, especially in blood.

In addition to the ELISA method other assays have become available including the use of immunofluoresence, radioimmuno-assay and Western blotting. Compared to these formats ELISA has the advantage of being simpler and less expensive but several problems are still evident. Use of enzyme labels requires an extra step in which the tests, usually carried out in microtitre wells, are incubated with enzyme substrate reagent in order to produce a coloured product. The colour reaction is allowed to proceed for a set time and the reaction is stopped using a strong mineral acid before reading the colour visually or photo-metrically. This step is prone to errors and adds significantly to the time needed to produce results. ELISA also suffers from the bulky, labile nature of the enzyme label, false results with whole blood and susceptibility to inhibition and denaturation.

More recently assays have appeared which replace enzyme label on the secondary antibody with conjugated gold label, these being referred to herein as 'immunogold assays'. The use of gold labelled secondary antibodies to the antibodies or antigens under assay allows visual identification of the antigen-antibody complex as a pink spot.

Whereas ELISA generally requires sophisticated equipment to measure substrate and product levels the immunogold test does not. Unlike the several hours often required to complete the ELISA, due to the substrate incubation step, the immunogold assay can provide results in as little as 10 minutes and thus prove of great assistance in the preparation of products such as Factor VIII where speed of extraction from freshly donated batches of blood is essential. Such immunogold assays are in use for example in the DuPont HIVCHEK (registered trade mark) and the Organon Teknika SPIA. The gold conjugate is relatively inexpensive, does not have the lability of enzyme labels and fewer manipulations are required over the ELISA assay.

The immunogold assays are not without problems of their own. The gold label provides a pink colouration as a positive result that is often very difficult to see. As ten percent of males have some colour blindness in the region of light concerned problems may arise in detection without the technician being aware of it. Furthermore, the test, as with ELISA, requires the use of serum or protein free fluids such as urine to function correctly.

Gold labelled antibodies have also been used in histology to stain entire cells and tissues so as to allow their structures to be seen under the microscope. In histology a technique for enhancing the gold stain by using the bound gold particles to catalyse local nucleation of silver particles from a solution of a silver salt has been developed which results in a much darker staining (see Danscher. G, Histochemistry 71. 81, (1981).

Holgate. C et al J. Histochem. Cytochem 31 938 (1983). The technique of silver enhancement (known in the art as 'immunogold silver staining' or 'IGSS') has problems such as self nucleation of silver which leads to deposition in areas other than those labelled with gold and the requirement that specific protocol is needed to achieve optimum results.

The use of such silver enhancement in immunogold blot overlay assays has been proposed in (1) EP-A-0158746 and (2) EP-A-0293947; (1) teaching prior purification of complex analytes (see (1) page 1, line 31-37) and (2) teaching use of filtration of samples (see (2) column 10, line 4 to line 38, Example 1.3, line 30 to 34) and neither teach suitability of the technique for whole blood sample assay. It is an object of the present invention to provide visually enhanced immunogold assays, permanent record immunogold assays and the capability to perform a reliable immunogold assay on a whole blood sample; the latter being unassayable by present ELISA techniques. Other objects and advantages of the present invention will be apparent to those skilled in the art on reference to the following specification and Examples.

The present invention provides a method for visually and/or photometrically and/or colorimetrically enhancing immunogold assays which employ specific binding agents immobilised onto solid substrates comprising exposing completed immunogold assay tests to a solution comprising silver ions whereby silver nucleates out onto at least a portion of the gold and then relating the presence and/or amount of nucleated silver to the presence and/or amount of the target analyte, characterised in that the assay is carried out on whole blood.

The present invention thus provides a first non-competitive enhanced immunogold assay option for the determination of the presence and/or quantity of a target analyte in whole blood comprising:
(a) immobilising onto a solid substrate a specific binding agent with which the target analyte will combine specifically
(b) exposing the immobilised specific binding agent to a sample of whole blood to be assayed
(c) exposing the immobilised specific binding agent from step (b) to a secondary binding reagent capable of binding to the specifically combined target analyte and to which colloidal gold particles have been bound
(d) exposing the immobilised specific binding agent from step (c) to a solution containing silver ions whereby the silver nucleates onto at least a portion of the gold bound to any bound secondary binding agent and
(e) relating the presence and/or amount of nucleated silver to the presence and/or amount of target analyte in the whole blood.

There is further provided a second, competitive, option for the performance of an enhanced immunogold assay of the invention, said option comprising:
(a) immobilising onto a solid substrate a specific binding agent with which the target analyte will combine specifically
(b) exposing the immobilised binding agent to a mixture of the whole blood to be assayed and a solution containing a quantity of target analyte, or an analogue thereof which will bind to said specific binding agent, to which colloidal gold particles have been bound
(c) exposing the immobilised binding agent from step (b) to a solution of silver ions whereby silver nucleates onto at least a portion of any gold bound to said binding agent via any labelled target analyte or analogue thereof and
(d) relating the presence and/or amount of nucleated silver to the presence and/or amount of target analyte in the whole blood to be tested.

A still further, competitive, option for the performance of an enhanced immunogold assay of the invention is provided comprising:
(a) immobilising onto a solid substrate a target analyte with which a specific binding agent will combine specifically
(b) exposing the immobilised target analyte to a mixture of the whole blood to be assayed and a specific binding agent with which the target analyte will combine specifically
(c) exposing the immobilised target analyte from step (b) to a secondary binding agent capable of binding to the combined specific binding agent and to which colloidal gold particles have been bound
(d) exposing the immobilised reference target analyte from step (c) to a solution containing silver ions whereby silver nucleates onto at least a portion of the gold bound to any bound secondary binding agent and
(e) relating the presence and/or amount of nucleated silver to the presence and/or amount of target analyte in the whole blood to be tested.

In each of the options the presence of the target analyte in the whole blood is determined by the presence or lack of silver deposit. In the first, non-competitive, option a positive result is provided by the presence of a silver deposit whereas the two competitive options show a reduced or no deposit as indicative of a positive result indicating that the labelled material has been prevented from binding by competition from analyte in the test blood. The amount of analyte may be determined from the degree of deposit, for example visually, colorimetrically or photo -metrically. Use of standard curves or colour charts for known analyte concentration absorbance values for the immobilised surface provides a convenient yet accurate measure of the amount of analyte in the test fluid. The invention is expected to be useful in enhancement of all types of whole blood immunoassays in which colloidal gold is used as a labelling agent and other options will occur to a man skilled in the art of binding assays.

The invention further provides kits for the performance of the method of the invention, said kits comprising substrate having specific binding reagent or target analyte immobilised thereon, a solution comprising silver ions, and either gold labelled secondary binding reagent or gold labelled target antibody.

The methods and kits of the present invention provide silver enhanced immunogold assays which at least partially overcomes the problems associated with the ELISA and immunogold assays previously described and provide a still higher degree of sensitivity with a absorbance improvement of 100 times being routinely obtained.

By use of a silver ion containing enhancer solution the gold is completely visualised to provide a dark brown to dense black deposit of metallic silver on the gold which may be produced in an amount proportional to the amount of gold labelled reagent bound to the solid-phase specific binding reagent. In the first option when the sample contains no target analyte then the secondary binding reagent gold conjugate cannot bind and no colour is produced thus giving a simple visual indication that no target analyte is present; the competitive assays giving a darkened negative. Results may alternatively be read visually using a conventional colorimetric plate reader with no filters being necessary.

As the silver layer is permanent the dried plates may be kept for future reading or checking. All the reagents are stable and the silver enhancement takes place under ambient conditions. An important advantage of the present method is its ability to assay whole blood as opposed to the prior assay's essential use of serum, plasma or non-blood liquid.

The method of the invention may be used to detect any target analyte in any aqueous fluid. Typical analytes include specific proteins, viruses, hormones, drugs, nucleic acids and polysaccharides; specificaliy antibodies, eg: IgD, IgG, IgM or IgA immunoglobulins to HTLV-I, HIV, Hepatitis A, B and non A/non B, Rubella, Measles, Human Parvovirus B19, Munps, Chicken Pox or Leukemia; human and animal hormones, eg: human growth hormone (hGH), human chorionic gonadotropin (hCG); drugs, eg: paracetamol or theophylline ; marker nucleic acids, eg; as for genetic finger printing analysis markers; polysaccharides such as cell surface antigens for HLA tissue typing and bacterial cell wall material; proteins such as carcinoembryonic antigen (CEA) or alpha -foetoprotein.

The whole blood may also be used undiluted but is preferably used diluted, eg: in the range 1:1 to 1:10, but dilution up to about 1:5000 is still found to yield useful results. A suitable diluent is a standard specimen diluent, eg: phosphate buffered saline.

In the assay of certain analytes, such as HIV antibody, there may be a degree of social sensitivity and the method of the invention advantageously enables assay of specimens in the form of samples blotted onto an absorbant material such as filter paper which can be allowed to dry and may be extracted using a suitable aqueous medium such as phosphate buffer saline; the dried sample may then be assayed as above. As little as 20 microlitres of whole blood need be blotted onto the absorbant material in this embodiment and extraction into typically 20 times this volume of aqueous medium provides a suitable extract for assay.

The solid substrate may be any material capable of having the specific binding reagent immobilised thereon; a preferred material being plastics or cellulose nitrate. The substrate may conveniently be of rod, bead or plate form. Most preferably the substrate is a microtitre plate, microwell strip or porous membrane and the specific binding reagent is immobilised, eg. into one or more of the wells on its upper surface, using techniques known in the art, such as those employed in conventional ELISA assay substrate preparation.

The specific binding reagent may be any material to which the analyte will bind specifically. It may, for example, be whole cells or tissue, cell fragments or protein, including protein which has been harvested from, or made by recombinant means from, an organism for which the infection thereby is being assayed for. For drugs the binding reagent may be an antibody specific for it. In the case of HIV the specific binding reagent may be the antigens which are commercially available and substrates already having such an antigen immobilised thereon are also commercially available, eg: as antigen coated microwell strips available from DuPont, as part of the DuPont HTLV-III/LAV ELISA Kit. When such commercially available substrates and immobilised antigen are used step (a) may be avoided in the practical performance of the assay.

The gold labelled secondary binding reagents may comprise any antibody binding to the target analyte and having colloidal gold particles conjugated to it. Preferably the gold particles are 10nm or less in size, 5 nm or less being the most effective. The exact nature of the secondary binding reagent may of course depend on the nature of the target antibody under investigation and suitable secondary antibodies will be apparent to those skilled in the art. Methods of conjugating colloidal gold particles to these secondary antibodies are known in the art and gold labelled secondary binding reagents are commercially available. These may be used in the method of the invention but preferably in a diluted state. A preferred concentration of gold labelled secondary binding reagent is that achieved by diluting the commercially available AUROPROBE LM GaHu (Mercia Diagnostics Ltd) in the range of 1:10 to 1:50; the 1:50 dilution proving to be most effective, Preferably other types of gold labelled reagent should be diluted to achieve htis level of concentration.

A wide range of anti-human, (eg: anti α, µ and γ), anti-rat, anti-mouse, anti-guinea pig and anti-rabbit antibodies which may be used as secondary binding reagents already labelled with gold are commercially available, eg: those available from Janssen Life Science Products under the registered trade mark AUROPROBE. For the purpose of testing human antibodies to most infections, eg HIV, the anti-γ agents which bind specifically to IgG are most useful as is Protein-A, a protein widely used for the detection of antibody molecules which is also available commercially bound to gold.

The silver ions in the solution used in step (d) may be supplied by any ionisable silver compound provided that the counter ion does not otherwise interfere with the assay. Preferred silver compounds are salts of organic acids, especially silver citrate or silver lactate, but any soluble inorganic silver salt, such as silver nitrate, may be used. The concentration of silver ion in the solution may vary greatly but is preferably in the range 0.1 to 1000 mM, more preferably from 1 to 100 mM. particularly from 2 to 20 mM. It is preferred to include a reducing agent, eg: an amino or hydroxybenzene such as hydroquinone, and a retardant for prevention of nucleation occuring too quickly, eg: gum acacia to the silver ion containing solution of step (d); the hydroquinone or gum acacia preferably being at 1 to 0.10% by weight.

The silver deposit may further be fixed onto the well using a suitable solution such as sodium thiosulphate although this is usually unnecessary. The preferred ingredients are selected to produce a light-stable assay relatively insensitive to water impurities with a large safety margin between adequate silver ion concentrations for the sensitivity of the assay and silver concentrations at which self-nucleation leads to unacceptably high background deposition. Suitable silver ion containing solutions for use in step (d) are commercially available. eg: for use in the IGSS methods described above as sold by Janssen under the registered trade mark IntenSE*II or by Sigma Chemical Co under the mark SE-100. A typical protocol for the first non-competitive option for performing the assay method of the invention is exemplified as applied to HIV as follows. Steps (a) and (b) may be carried out in a manner identical to that used in the known ELISA methods, except that whole blood, preferably diluted with phosphate buffered saline (PBS) is used as the fluid. It is preferred to expose the binding reagent, ie: the antigen, first to PBS containing goat serum for about two minutes to prevent non-specific binding at lower concentrations of goat serum than would otherwise be used.

The method is found to work at serum dilutions in the range 1:1 to 1:1600 with no suggestion that dilutions outside these limits would result in failure. Typically the whole blood is incubated with the immobilised antigen up to 30 minutes at 37°C but times of 15 minutes may be used. After step (b) excess whole blood is removed by known methods, eg: washing with PBS.

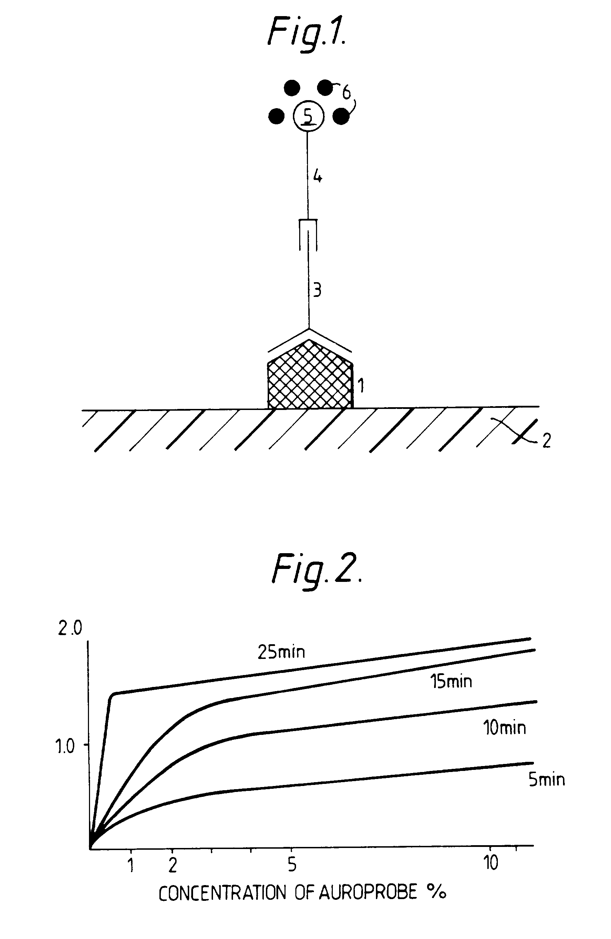

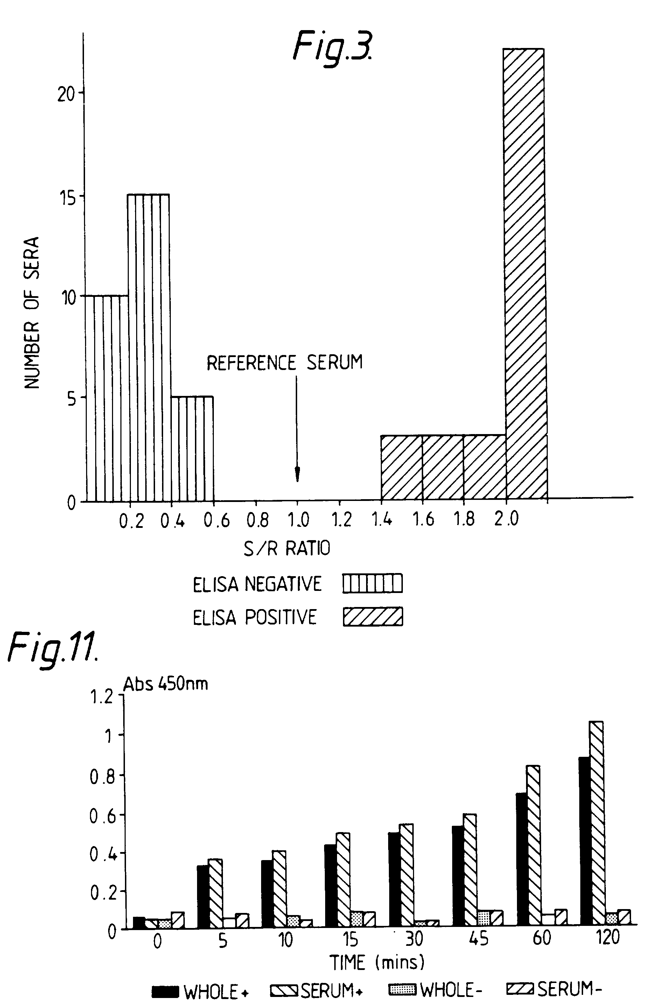

The gold labelled binding agent is preferably incubated with the combined unlabelled agents for 10 to 60 minutes, more preferably 20 to 40 minutes and most preferably 30 minutes. Step (c) of the first option is typically carried out by incubating the binding reagent, eg: antigen, with a solution containing gold labelled secondary binding reagent, eg:secondary antibody, eg: containing 5% AUROPROBE, gelatin (eg: 5% to minimise non-specific binding) and 0.1% goat serum in PBS, at 37°C for 15 minutes. This protocol is an optimum for use with a substrate in the form of microtitre wells containing a coating of 300ng of IgG. The incubation period does not appear to be critical between 5 and 30 minutes at high anti-HIV titres but low HIV positive sera need about 10 minutes incubation to achieve maximum binding. Fig 2 of the accompanying drawings shows the effect of different AUROPROBE concentrations and incubations with respect to the final absorbance of the wells after silver enhancement. After step (c) excess gold labelled antibody solution is removed by known methods, eg: washing with demineralised water.

The silver ion exposure steps (d) and (e) are typically carried out by exposing the immobilised specific binding agent. eg: antigen, to a solution of silver ions, eg: IntenSE*II made up to suppliers specifications at room temperature for 15 minutes, preferably with shaking. The time required to reach maximum absorbance in an anti-HIV assay is found to be dependent on the anti-HIV titre. During the first 20 minutes or so the silver precipitation process is specific for gold particles, thereafter self nucleation begins to occur. The silver solution is thus preferably incubated with the completed immunogold assay for 10 to 60 minutes, more preferably 20 to 30 minutes.

As mentioned above the presence of the target analyte in the first option is manifested by the appearance of a visible brown or a black colouration, the intensity of which may be quantitatively measured by absorbance measurement or comparison with standard colour charts. It is preferred to carry out multiple assays and to compare these with standards and controls. The specific volumes, etc, used in the assay method of the invention will of course vary with the particular circumstances, eg: the nature of the substrate, but is adaptable to work on a microlitre scale using microlitre well substrates. The kits for performance of the method of the invention may be provided in a number of forms and ways of adapting immunoassay methods into kit form are well known. The invention will now be illustrated by way of example only with reference to the Figure 1, which shows the chemical basis of the method as applied to HIV in the first non-competitive option ; Fig 2, which shows graphically the effect of increasing AUROPROBE percentages and step (c) incubation times; Fig 3. which shows test results and Fig 4, which shows the effect of dilution of the serum sample; Figs 5 and 6, which show the absorbance at 450nm of assay microtitre wells of ELISA and SEGLISA assays carried out upon the same HIV whole blood samples; Figs 7 and 8, which show histograms of 50 positive and 50 negative HIV sample results from a SEGLISA; Figs 9 and 10, which show compared results from assays run with whole blood and serum samples for HIV antibody assay; Fig 11, which shows a histogram for the effect of incubation time of the sample in the microtitre wells with serum and whole blood; Fig 12 shows a schematic diagram of the binding basis for a SEGLISA assay for theophylline; Fig 13 which shows the binding basis for a SEGLISA assay for Rubella antibodies; Figs 14. to 21, which show the effect of gold conjugate dilution, gold particle size, incubation time with gold conjugate, gold conjugate solution volume, amount of gelatin in the conjugate solution, type of silver enhancer kit used, incubation time with silver enhancer and volume of silver enhancer on the assay result as measured by microwell absorbance.

Referring to Fig 1, the principle of the enhancement method is shown with regard to the first option. An antigen (1) is immobilised onto a solid polystyrene substrate (2). A human anti-HIV antibody (3) has been captured by the antigen (1) and is therefore itself immobilised on the substrate (2). The antibody (3) has been exposed to an anti-human IgG (4) labelled with a coloidal gold particle (5) and thus labelled IgG (4) has become bonded to the antibody (3). The antigen -antibody-IgG (1)(3)(4) complex has been exposed to a solution containing silver ions and these have become nucleated around the gold particles (5) as solid silver particles (6) having a dark colour to the eye.

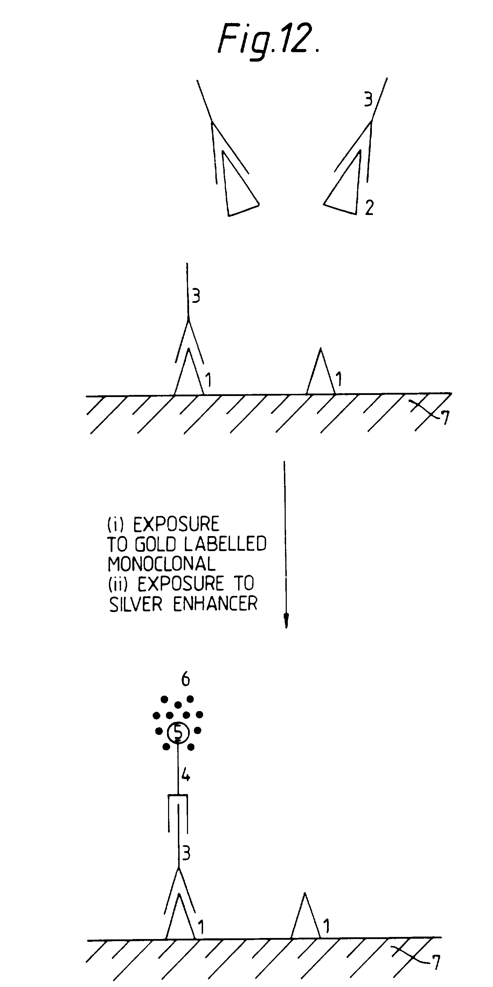

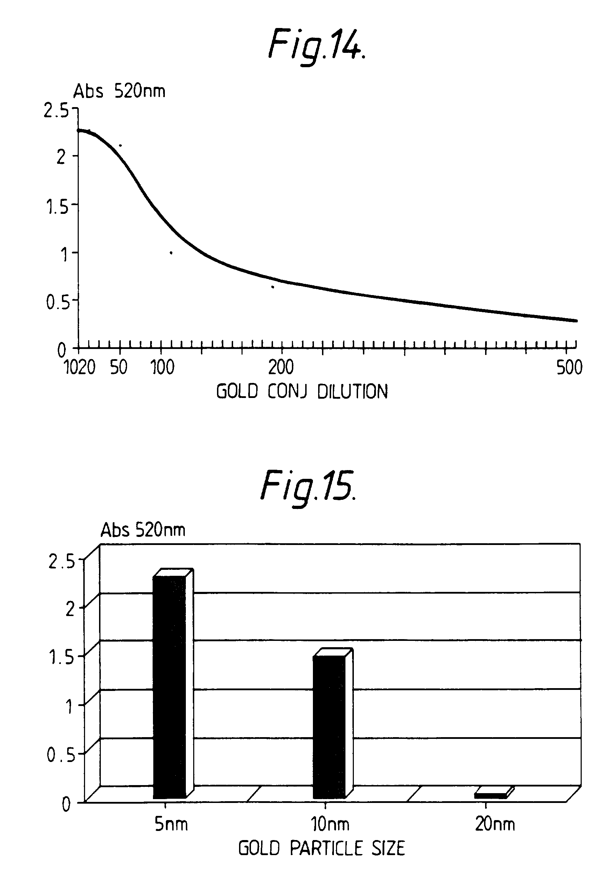

In Figure 12 (1) is theophylline, (2) is sample theophylline, (3) is anti-theophylline monoclonal antibody, (4) is antimouse IgG-gold, (5) the gold in that, (6) silver deposit and (7) wells. In Figure 13 is a schematic diagram of the mechanism of this assay wherein (1) is bound anti-hGH, (2) is hGH, (3) is monoclonal antibody to hGH, (4) is anti-IgG-Gold conjugate, (5) is the gold label in that conjugate, (6) is silver deposited onto said gold and (7) is the well surface.

The term SEGLISA is used herein to indicate use of the present Silver Enhanced Gold Labelled Immunosorbant Assay. The method of the invention will now be further illustrated by way of the Examples from which it will be clear to a man skilled in the art that many other possibilities may be derived for assay of other target analytes within the scope of the invention.

### COMPARATIVE EXAMPLE 1. SEGLISA ASSAY OF HIV IN HUMAN BLOOD SERUM.

Materials: Except where otherwise stated laboratory reagents were obtained from BDH Ltd, Poole, Dorset, BH12 4NN. Antigen-coated microwell strips were a gift from DuPont (UK) Ltd, Stevenage, UK. and are usually sold as part of the DuPont HTLV-III/LAV ELISA kit.

The gold and silver reagents were supplied by Janssen Life Sciences, Wantage, Oxon, OX12 ODQ. These consisted of goat anti-human IgG labelled with colloidal gold particles of 10nm mean diameter (AUROPROBE BL plus GAHu) and IntenSE*II (product No 30.653.01), a silver enhancement reagent kit that works well under ambient light conditions. Both reagents still worked well after one year's storage; the AUROPROBE at 4°C and the IntenSE*II at room temperature. AUROPROBE and IntenSE*II are Janssen registered trade marks. Specimens: In order to assess the new assay a panel of samples consisting of known anti-HIV positives and negatives were compiled. The negative sera were obtained from specimens sent for tests other than HIV testing. The positives were from patients being investigated or treated for HIV infection, persistent generalised lymphadenopathy, AIDS related complex or AIDS. As a preliminary to this study all the samples in the panel were tested and categorised as being either positive or negative. this involved screening by a commercial ELISA kit and confirmation of positive results by the Virus Reference Laboratory (Colindale Avenue, London) using complementary tests. The Central Public Health Laboratory Division of Microbiological Reagents and Quality Control (DMRQC) supplied six plasma controls (panel No 8) consisting of five positives, prepared by diluting a high titre positive specimen, and one negative. Another DMRQC control preparation (designated LP3), which was a positive sample diluted to the point where it gave neither a strong positive or negative reading, was used as a reference serum.

**Apparatus**: Standard laboratory ELISA equipment was used. The absorbance of the microtitre wells was read using a Dynatech MR250 (registered trade mark) plate reader fitted with a 450nm filter. A special grade 2 microbiological safety hood was used.

**Protocol**: One hundred microlitres of phosphate buffer saline (PBS) (0.1 M NaCl, 2.7 mM KCl, 1.5 mM KH₂PO₄, 8.1 mM Na₂PO₄, pH 7.3) containing 10% of normal goat serum and 0.5% Tween 20 (registered trade mark) was added to each well of a standard ELISA microtitre well plate and the plates left to stand for a minimum of 2 minutes at room temperature. 50 microlitres of the PBS was then removed from each well and 50 microlitres of test serum (or where appropriate reference serum or PBS) was then added. Each strip would typically contain one reference and one blank well. The wells were sealed, agitated for 10 seconds and then incubated at 37°C for 15 minutes before being washed five times with 300 microlitres of PBS containing 0.1% goat serum. 50 microlitres of the gold conjugate reagent was then added (this solution cotains 5% AUROPROBE, 5% gelatin and 0.1% goat serum in PBS). The wells were sealed and agitated as before and then incubated at 37°C for 15 minutes. After washing again as above with demineralised water (3 x 300 microlitres), 200 microlitres of a freshly made up solution of silver enhancer solution (made up by mixing equal volumes of the two solutions comprising the IntenSE*II kit) was added to the wells and the wells were shaken at room temperature for 15 minutes. The absorbance of the wells was then measured and known negative and positive controls were included in each batch.

HIV positive samples produced a thin black high-contrast coating on the well bottom and sides (absorbance typically greater than 1) while very dilute positive sera gave a brown discolouration to the wells. With negative sera there was no visible staining.

Development of silver enhancement: Experiments to discover the effects of variables on the time-consuming incubation stages were carried out with the aim of designing a batch assay capable of producing results in a relatively short time. Some observations are summarised below.

**Antigen/anti-HIV reaction**: Neat test serum was diluted by adding it to microwells containing an equal volume of buffer. Goat serum in the buffer was necessary to prevent non-specific binding, however, it also decreased the rate of reaction. It was found that by adding the buffer containing the goat serum before the test serum (at least 2 minutes) that non-specific binding was prevented using a lower concentration of said goat serum than would otherwise be the case.

Decreasing the incubation time from 30 minutes to 15 minutes produced no decrease in absorbance thus it may be possible to decrease the reaction time further but the need for precise timing between manually loading each well and reading it makes very short incubation periods more difficult to cope with.

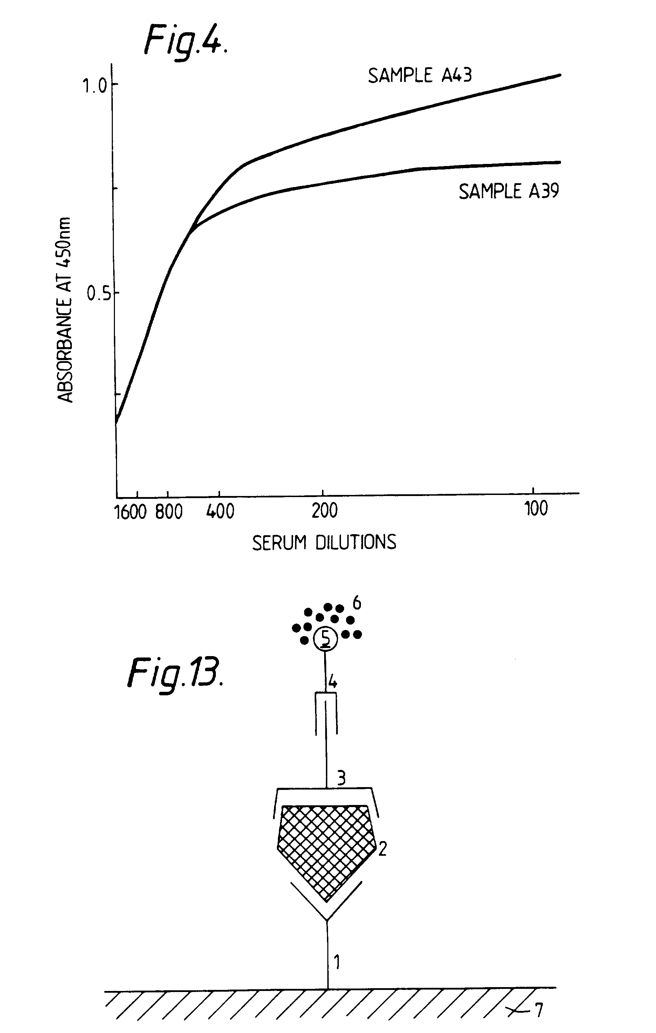

**Results**: A panel of 61 samples comprising 30 HIV negative and 31 HIV positive samples was tested by SEGLISA. The DMRQC control designated LP3 was used as a reference (cut-off standard). The results are presented in terms of the ratio of sample absorbance/reference absorbance. This S/R ratio is equal to 1 when the absorbance of test sample equals that produced by the reference serum. The test ratios are illustrated graphically in Fig 3.

Positive and negative sera fell into two clearly discernable groups. For the negative sera the mean S/R was 0.31 while for the positives this was 2.72. Mean positives/mean negatives gave a value of 8.66. The DMRQC controls were also correctly categorised (ie: one negative and five positives).

As with all anti-HIV test procedures the sensitivity and specificity of the assay is influenced by the chosen levels of the cut-off values; unfortunately there are no absolute standards. As the threshold for defining the minimum positive SEGLISA ratio decreases the sensitivity of the assay in detecting weak anti-HIV cases increases while the specificity decreases. Since the positive results found by a screening test would normally be confirmed by several other tests of different formats it is best to bias the cut-off towards producing false positives rather than false negatives. For example, for the present assay we would suggest a cut-off ratio of 0.8 for use in blood screening. At this ratio the cut-off should not unnecessarily exclude large quantities of donated blood.

If we define sensitivity of the assay as the proportion of the positive sera correctly identified then the test has a sensitivity of 100%; if the assay specifically is defined as the proportion of negatives correctly identified then the specificity was 100%.

**Analytical sensitivity**: The ability of the assay to detect low concentrations of HIV antibodies was assessed by titrating two anti-HIV positive sera. The positive sera were dilutued in negative serum to give dilutions of 1:100, 1:200, 1:400, 1:800 and 1:1600. At dilutions of 1:1600 both sera still gave positive results. As can be seen from Fig 4 the slope of the curve is steepest at high sample dilutions. This gives maximum sensitivity to the detection of sera with very low antibody titres.

### EXAMPLE 1. SEGLISA ASSAY OF HIV IN WHOLE HUMAN BLOOD.

**Methods and materials**: HIV antigen coated wells and positive and negative HIV antibody controls were supplied by Mercia diagnostics Ltd, Anti-HuIgG conjugate (AUROPROBE LM GaHu) was supplied by Janssen Life Sciences, Anti-HuIgG-Horse radish peroxidase and silver enhancing solution (SE-100) and all other reagents were supplied by Sigma Chemical Company. Storage of reagents was at 4°C except for raw chemicals for silver ennancement solutions which were stored at room temperature in the dark. All experiments were carried out in duplicate with results quoted as the mean absorbances between the two. The cut-off value for a positive and a negative HIV antibody level was taken as the mean absorbance of the positive HIV antibody control divided by two. Absorbances of tests above this value were termed positive for HIV antibodies and below this value as negative for HIV antibodies.

**Specimens**: A panel of 50 known anti-HIV positive samples were collected from blood specimens sent into the laboratory from patients suffering from HIV infection. 50 blood samples were also collected from specimens sent into the laboratory for other biochemical analysis.

**Apparatus**: Standard laboratory micro-titre equipment was used. All testing was carried out under a special grade 2 micro--biological safety hood. The absorbance of the micro-titre wells was read using a Dynatech MR250 plate reader fitted with a 450nm filter.

**Assay procedure**: Test sera and controls were diluted 1 in 20 and test whole blood 1 in 10 with specimen diluent (8g NaCl, 0.2g KH₂PO₄, 2.9g NaH₂PO₄.12H₂O + 1% BSA + 0.05% Tween 20 made up to 1 litre using distilled water), 200 microlitre of each was added to micro-titre wells pre-coated with inactivated HIV antigens, the wells were incubated at 37°C for 60 minutes. The serum or whole blood was then aspirated off and the wells were washed 5 times in wash buffer (8g NaCl, 0.2g KH₂PO₄, 2.9g NaH₂PO₄.12H₂O +0.05% Tween 20 made up to 1 litre using distilled water). The antibody /antigen complex could then be quantified by either ELISA or SEGLISA.

SEGLISA: 100 microlitres of IgG-Gold conjugate (diluted 1 in 50 with specimen diluent) was added to each of the wells, incubation was again at 37°C for 60 minutes. The wells were then washed 3 times with wash buffer and silver enhanced (100 microlitres of equal volumes of initiator A and enhancer B) for 30 minutes at room temperature. After washing 3 times in distilled water the absorbances of the wells were read at 450nm.

Comparative ELISA: 100 microlitres of Anti-HulgG-Horse radish peroxidase conjugate (diluted 1 in 200 with specimen diluent) was added to each well and again incubated at 37°C for 60 minutes. The conjugate was aspirated off and the wells were washed 3 times in wash buffer. The substrate was made up 10 minutes prior to use by diluting Tetramethylbenzidine chromogen (TMB) 1 in 100 with substrate solution (citrate buffer + H₂O₂). This was incubated on the wells at room temperature for 30 minutes after which time 25 microlitres of 2 M H₂SO₄ was added to stop the reaction. The wells were then read within 30 minutes for absorbance at 450nm. Results: 15 blood samples shown to be negative for HIV antibodies were tested using serum and whole blood using the ELISA and SEGLISA methods descibed above. Table 1 shows a summary of the results obtained.

**TABLE 1.**

| Assay | Serum | | Whole blood | |
|---|---|---|---|---|
| | + | - | + | - |
| Cut off Abs. | 0.213 | 0.210 | 0.388 | 0.378 |
| ELISA | 0 | 15 | 12 | 3 |
| SEGLISA | 0 | 15 | 0 | 15 |

+ = a positive result; - = a negative result. Cut-off value for positive and negative results is the mean absorbance of the positive control divided by two. Readings were at 450nm.

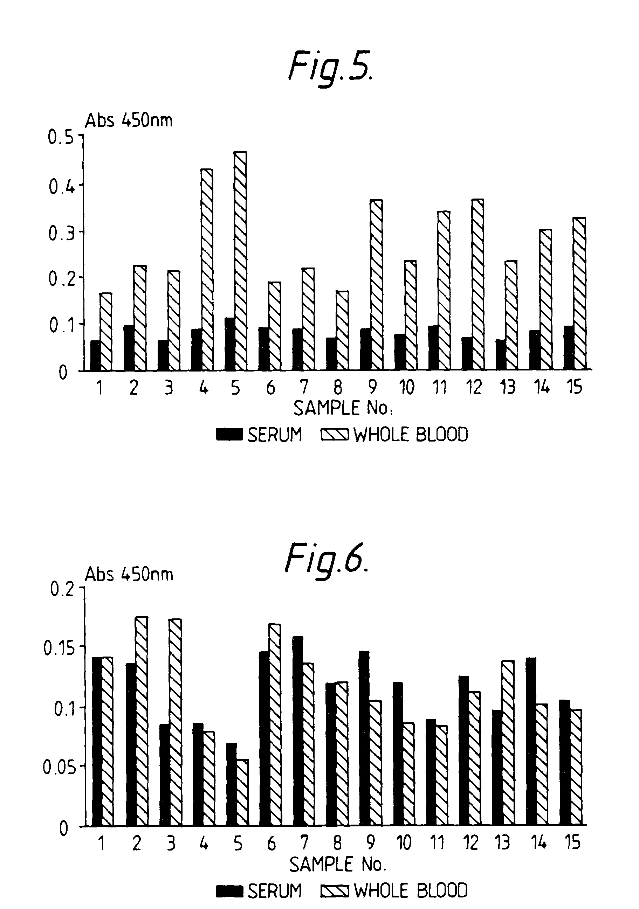

Figures 5:ELISA and 6:SEGLISA show this result in more detail. The ELISA correctly assigned all 15 samples negative when using the serum, however when using whole blood 12 samples were given as falsely positive, with all 15 showing absorbances at least double those obtained from equivalent serum samples. The SEGLISA correctly identified all 15 samples as being negative for HIV antibodies when using both serum and whole blood methods. These results show that a HRP-ELISA is unsuitable to test whole blood specimens, whereas SEGLISA reliable results unaffected by the presence of the red cells.

50 known positive HIV Ab and 50 known negative HIV Ab whole blood samples were tested by a SEGLISA. Table 2 shows the results obtained.

**TABLE 2.**

| Positive batch | Positive | Negative |
|---|---|---|
| No. of samples | 50 | 0 |
| Negative batch | | |
| No. of samples | 0 | 50 |

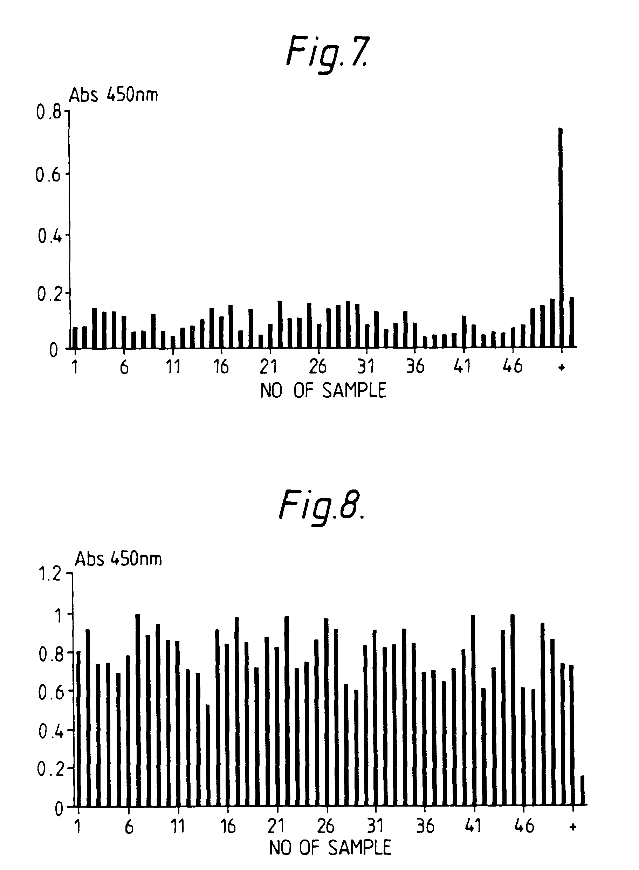

Cut-off value for positives was 0.362 and for negatives was 0.368. Figures 7 and 8 show histograms of the negative and positive screen respectively. All 50 negative patient samples give an absorbance of below 0.362 and all 50 positive samples give an absorbance above the cut-off value of 0.368.

**Precision**: A positive HIV Ab whole blood and a serum sample were assayed by a SEGLISA 16 times. All aspects of the assay were kept constant. The results from these precision tests are shown in Table 3.

**TABLE 3.**

| | Serum | Whole blood |
|---|---|---|
| Number of entries | 16 | 16 |
| Mean | 0.502 | 0.539 |
| SD | 0.044 | 0.041 |
| C of V | 8.7% | 7.5% |

The mean given is an average absorbance of all the individual results, SD is the standard deviation of the set of results and C of V is the correlation coefficient. The whole blood SEGLISA gives a very respectable C of V of 7.5% which indicates a good precision. It is also comparable to the serum SEGLISA which gives a C of V of 8.7%, suggesting that the whole blood does not have a varied effect on the assay.

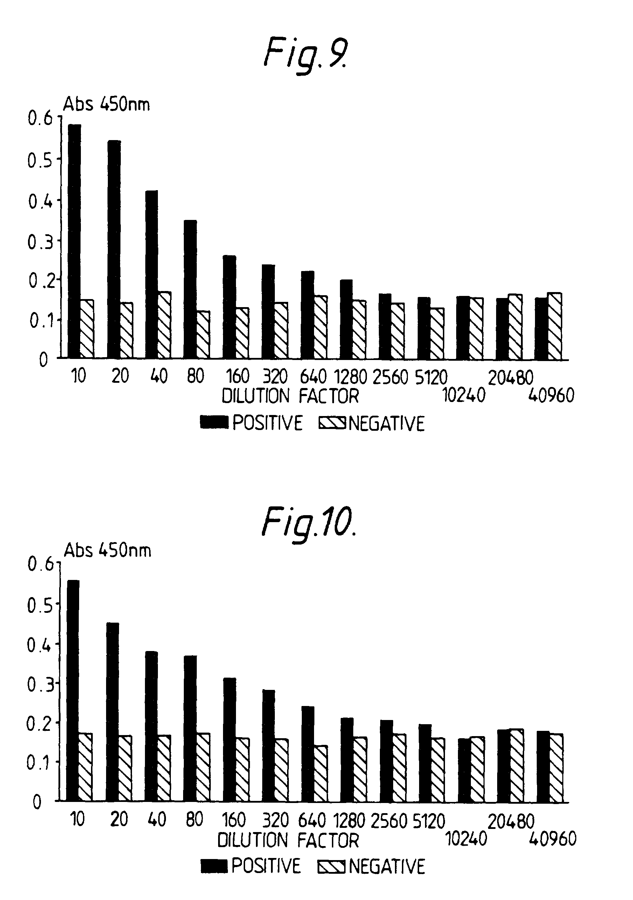

Sensitivity: The sensitivity of the assay was measured by using various titrations of a positive and negative anti-HIV whole blood sample and comparing the results with the equivalent serum test. Serum was diluted 1:10, 20, 40, 80, 160, 320, 640, 1280, 2560, 5120, 10240, 20480 and 40960 with specimen diluent. The blood cell fraction of a negative HIV Ab sample was used to add to each dilution to keep the serum/blood cells ratio at 1:1 whilst maintaining the quoted dilutions, with PBS buffer added for the equivalent serum assay. Figure 9 shows the results obtained from the whole blood assay, on dilution of the positive sample we see that it becomes negative at 5120 dilution factor. These results are comparative with the serum assay, as shown in Figure 10, which becomes negative at a serum dilution factor of just above 5120. These results show that sensitivity is not lost when using whole blood.

**Incubation times of whole blood**: A positive and a negative HIV Ab sample was tested as whole blood and as serum with varying incubation times: 0, 5, 10, 25, 30, 45, 60 and 120 minutes were tested. The whole blood was also observed to record it's appearance in the wells after each incubation time as shown in Table 4.

**TABLE 4.**

| Minutes | State |
|---|---|
| 5 | Totally mixed. |
| 10 | Totally mixed. |
| 15 | Totally mixed. |
| 30 | Slight serum layer at top fifth of blood. |
| 45 | Diffuse serum layer at top third of blood. |
| 60 | Diffuse serum layer at top half of blood. |
| 120 | Layer of cells at bottom fifth of blood. |

Figure 11 shows a histogram of the results obtained. An increase of absorbance is seen as incubation times increase, with serum giving slightly higher absorbances than whole blood. It is therefore shown that the separating affect of cells and serum/buffer during incubation on wells, has no significant effect on the immunological reaction.

### EXAMPLE 2. SEGLISA ASSAY OF HIV IN WHOLE BLOOD SAMPLES BLOTTED ONTO FILTER PAPER.

A test was developed to see if antibodies could be extracted from whole blood that had been spotted onto an absorbant carrier material, eg:filter paper, with the aim of providing a 'far station' test submission procedure whereby patients could submit samples by post; as there is no requirement for the analyte being determined to be a viable part of the infection any characteristic compound may be assayed.

Guthrie-PKU test filter paper was used as the spotting medium. 1 cm squares were cut out and placed at the bottom of 5 ml tubes. 0.1 micrograms/ml solution of IgG labelled with I¹⁵ was diluted 1:10 using whole blood. 20 microlitres was then added to each of 5 tubes leaving one blank. All 6 tubes were immediately counted for gamma emission on a NE 1600 gamma counter for 120 seconds. The tubes were then placed into a 37°C incubator for 60 minutes to dry. After this time 500 microlitres of PBS-BSA-Tween was added to each tube and vortex mixed for 1 minute before being agitated for 15 minutes. The buffer was then aspirated off and placed in a separate tube, both filter paper and buffer were then counted for gamma emission for 120 seconds. Results are displayed in Table 5.

**TABLE 5.**

| Tube | Blank | 1 | 2 | 3 | 4 | 5 | Average |
|---|---|---|---|---|---|---|---|
| Totals | 98 | 1282 | 1229 | 1239 | 1269 | 1248 | 1248 |
| Filter | 103 | 261 | 241 | 218 | 247 | 207 | 235 |
| Buffer | 143 | 960 | 992 | 1096 | 1089 | 1021 | 1032 |

Percentage bound is 100%
After extraction percentage left on the filter paper = 235/1248 x 100 - 18%
After extraction percentage left in the buffer = 1032/1248 x 100 = 82%

A G-75 Sephadex column was used to separate out the IgG tracer used in the experiments and it was found that 78% of the tracer was bound to the antibody. 80% of the counts had been extracted from the filter paper thus the proportion of the antibody extracted is found by 58/(58+20) x 100 = 74%. Having shown the viability of extracting the antibody a SEGLISA was carried out as follows:

Guthrie-PKU test filter paper was used as a spotting medium whereby 1 cm squares were cut out and placed at the bottom of 5 ml tubes and whole blood samples positive for HIV antibodies and 15 whole blood samples negative for HIV antibodies were tested against controls of positive and negative HIV antibody serum. 20 microlitres of each sample was then spotted onto the filter papers in the tubes. All the tubes were then sealed and allowed to stand overnight to dry. After this time 400 microlitres of PBS-BSA-Tween was added to each tube and mixed on a sero-agitator for 30 minutes. 200 microlitres of the buffer was then aspirated off and placed in a HIV antigen coated well in order to carry out a SEGLISA by the method of Example 2. with results as set out in Table 6.

**TABLE 6.**

| Specimen | 1 | 2 | 3 | 4 | 5 | 6 | 7 |
|---|---|---|---|---|---|---|---|
| Positive Abs₄₅₀ | 0.610 | 0.721 | 0.511 | 0.492 | 0.811 | 0.510 | 0.595 |
| Negative Abs₄₅₀ | 0.127 | 0.134 | 0.114 | 0.115 | 0.125 | 0.099 | 0.142 |
| Specimen | 8 | 9 | 10 | 11 | 12 | 13 | 14 |
| Positive Abs₄₅₀ | 0.611 | 0.734 | 0.131 | 0.710 | 0.739 | 0.512 | 0.687 |
| Negative Abs₄₅₀ | 0.090 | 0.152 | 0.131 | 0.115 | 0.110 | 0.106 | 0.113 |
| Specimen | 15 | Control | | | | | |
| Positive Abs₄₅₀ | 0.743 | 0.788 | | | | | |
| Negative Abs₄₅₀ | 0.121 | 0.138 | | | | | |

All results have been corrected by a background factor of 0.109 derived from the present and previous means of the negative results.

### EXAMPLE 3. OPTIMISATION OF PARAMETERS FOR SEGLISA.

In order determine optimal parameters for the SEGLISA assay standard experiments were carried out using microtitre plates coated with Human ImmunoglobulinG (HuIgG) to bind antibody-gold -conjugate specific for it and then enhancing this using a variety of enhancing conditions.

**Methods and materials**: Microtitre plates were supplied by Greiner Co. Protein A-Gold conjugate and SE-100 silver enhancement kit was supplied by Sigma Chemical Co in 5, 10 and 20 nm gold particle sizes. Anti-HuIgG-Gold conjugate (AUROPROBE GAHu G5) and IntenSE M and IntenSE BL silver enhancement kits were supplied by Janssen Life Science Products Ltd. All other chemicals unless stated were supplied by Sigma Chemical Co. Storage of chemicals and reagents was at 4°C for all except the neat Protein A-Gold conjugate which was stored at -20°C. All tests were carried out in duplicate and average absorbances were used.

**Coating of microtitre plates with HuIgG**: A 96 well microtitre plate was coated with purified Human Immunoglobulin G (HuIgG). 1mg of HuIgG was dissolved in 1 ml of coating buffer (1.59g Na₂CO₃, 2.93g NaHCO₃, 0.2g NaN₃ made up to 1 litre with demineralised water and adjusted to pH 9.6). The 1 mg/ml stock solution was diluted further to give the desired HuIgG concentrations. The absorbance of the HuIgG solution was read at 280nm. 200 microlitres of the solution was added to each well and the plate was left overnight to incubate at 4°C. After incubation the absorbance of the HuIgG solution from the wells was read again at 280nm. Calculations of the amount of Human IgG absorbed to the plate gave a range of between 200 and 300ng HuIgG per well. The plate was washed with PBS + 1%BSA(8g NaCl, 0.2g KH₂PO₄, 2.9g NaH₂PO₄.12H₂O + BSA made up to 1 litre using demineralised water) three times then left for one hour with 250 microlitres PBS + 1%BSA per well at room temperature. After this time the plate was washed again three times with PBS + 1% BSA + 0.5%Tween 20 (8g NaCl, 0.2g KH₂PO₄, 2.9g NaH₂PO₄.12H₂O + 1% BSA + 0.5%Tween made up to 1 litre using demineralised water) and allowed to dry. The plate was then immediately stored at 4°C.

**SEGLISA**: 50 microlitres of protein-A conjugate diluted with PBS + 1% BSA . 0.5% Tween was added to each well and incubated at room temperature for 20 minutes. The plate was then washed three times with PBS + 1% BSA . 0.5% Tween 20 and then allowed to dry. 200 microlitres of freshly made up silver enhancer solution (equal volumes of Enhancer A and Initiator B) was added to each well and the wells left for 15 minutes. After this time the enhancer was washed off with demineralised water and the wells were immediately read on a plate reader at 520nm.

**RESULTS**: In order to optimise the SEGLISA various parameters were studied using constant values for parameters not under study. The results of the studies are summarised in the Figures 14 through 23. These results provide the following findings:

**Figure 14**: Shows Absorbance values for a SEGLISA performed as above using various dilutions of gold conjugate from 1:10 to 1:200 in PBS + 1%BSA + 0.5% Tween 20. The plot shows a plateau between dilution factors of 10 to 50 whereby it can be seen that a dilution factor of 50 is the most efficient.

**Figure 15**: Shows Absorbance values using various particle sizes for the gold in the conjugate whereby it is seen that 5nm gold particles give the highest absorbance whereas 20nm gold particles give virtually no absorbance.

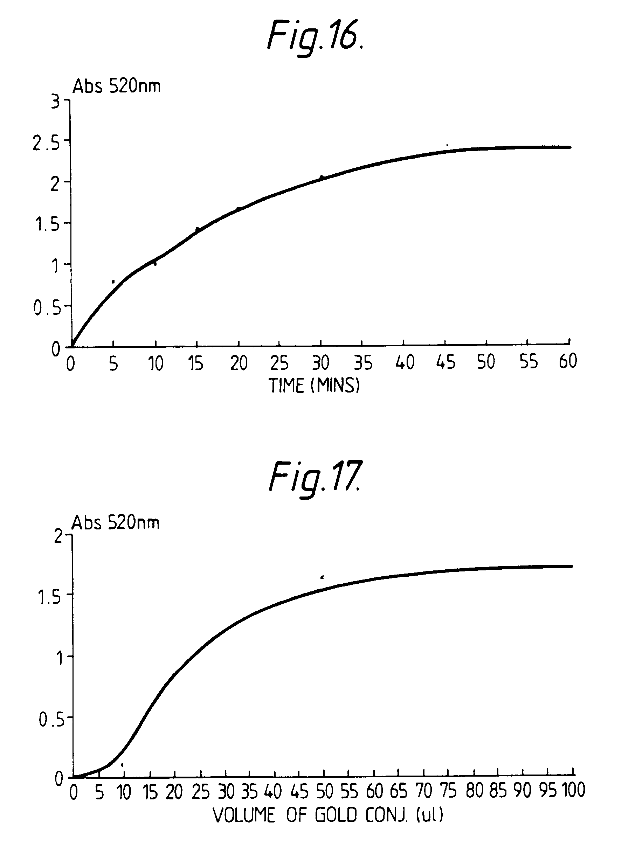

**Figure 16**: Shows absorbance values using various incubation times with the gold conjugates from 0 to 60 minutes: 30 minutes proving to be the optimum time.

**Figure 17**: Shows absorbance values using various volumes of gold conjugate solution from 0 to 100 microlitres. 5, 10 and 20 microlitres volumes were not sufficient to cover the whole of the bottom of the flat microtitre well; 50 and 100 microlitre volumes give high absorbances with the wells being fully covered resulting in an even conjugate and thus even silver deposit.

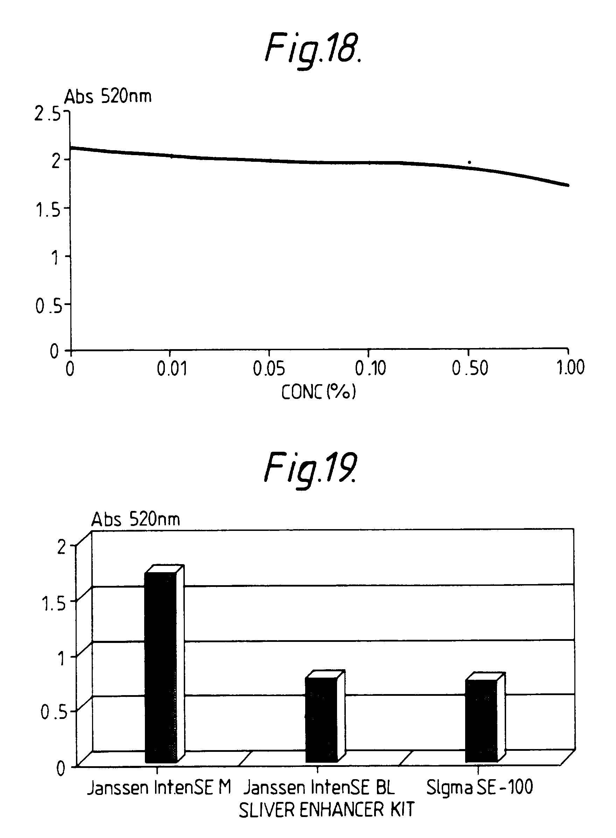

**Figure 18**: Shows the effect of varying the-amount of gelatin in the conjugate solution (as a colloid stabiliser); no significant difference was found for gelatine additions of 0 to 0.5%.

**Figure 19**: Shows the absorbance obtained using equivalent samples with SEGLISAs using three different silver enhancement kits; IntenSE M gave the highest absorbance with a mirrored black deposit. The Sigma kit did give a mirrored black deposit of satisfactory nature when stabilised with hydroquinone and gum acacia as used in the other Examples herein.

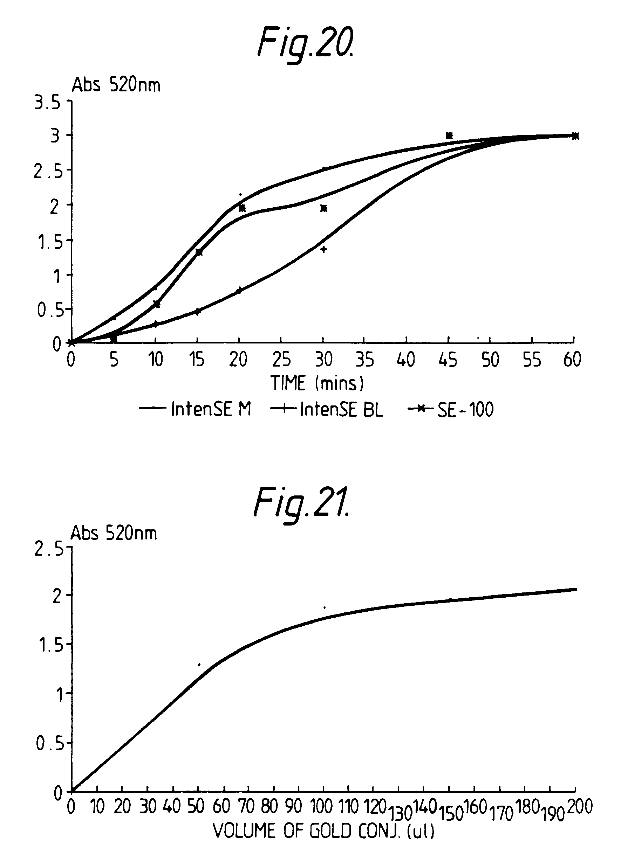

**Figure 20**: Shows the effect of incubation time with the silver enhancer for each of the kits; 20 to 30 minutes provides a high enough absorbance to work with before self nucleation sets in.

**Figure 21**: Shows a steady increase in absorbance as volume of enhancer is increased up to 100 microlitres.

### EXAMPLE 4. SUITABLE LABORATORY PREPARED SILVER ENHANCER REAGENT.

In order to provide a non-commercially available, laboratory preparable alternative silver enhancer solution experiments were carried out on the HIV immunogold procedure and the following reagent kit was found to be suitable comprising four reagent solutions:
(1) Solution A: Protective colloid gum acacia, 50g in 100ml of distilled water.
(2) Solution B: Citrate buffer 23.5g Tri-Na citrate-2 hydrate and 16.36g anhydrous citric acid in 100ml of distilled water.
(3) Solution C: Reducing agent hydroquinone 0.85g in 15ml of distilled water (prepared just prior to use).
(4) Solution D: Silver lactate 0.11g in 15ml distilled water (prepared just prior to use).

The four solutions were mixed together before use and tnen stored in the dark. The ratio of each solution in the final enhancer solution was: Solution A - 7.5ml made up to 60ml with distilled water; Solution B - 10ml; Solution C - 15ml; Solution D - 15ml. 100 microlitres of the mixed solution was added to each well in the dark and allowed to incubate for 15 minutes before washing three times with distilled water.

## Claims

1. A method for visually, and/or photometrically and/or colorimetrically enhancing immunogold assays which employ specific binding agents immobilised onto solid substrates and which are carried out upon whole blood comprising exposing completed immunogold assay tests to a solution comprising silver ions, whereby silver is nucleated onto at least a portion of the gold, and then relating the presence and/or amount of nucleated silver to the presence and/or amount of the target analyte.

2. A method for the determination of the presence or quantity of a target analyte in whole blood comprising:
(a) immobilising on a solid substrate a specific binding agent with which the target analyte will combine specifically
(b) exposing the immobilised specific binding agent to a sample of whole blood to be assayed
(c) exposing the immobilised specific binding agent from step (b) to a secondary binding agent capable of binding to the specifically combined target analyte and to which colloidal gold particles have been bound
(d) exposing the immobilised specific binding agent from step (c) to a solution containing silver ions whereby the silver nucleates onto at least a portion of the gold bound to any bound secondary binding reagent and
(e) relating the presence and/or amount of nucleated silver to the presence and/or amount of target analyte in the whole blood.

3. A method for the determination of the presence and/or quantity of a target analyte in whole blood comprising:
(a) immobilising on a solid substrate a specific binding agent with which the target analyte will combine specifically
(b) exposing the specific binding agent to a mixture of the whole blood with a solution containing a quantity of the target analyte, or an analogue thereof which will specifically bind to said specific binding agent, to which colloidal gold particles have been bound
(c) exposing the immobilised specific binding agent from step (b) to a solution comprising silver ions whereby silver nucleates onto at least a portion of any gold bound to said binding agent via any labelled target analyte or analogue thereof and
(d) relating the presence and/or amount of nucleated silver to the presence and/or amount of target analyte in the whole blood.

4. A method for the determination of the presence and/or quantity of a target analyte in whole blood comprising:
(a) immobilising on a solid substrate a target analyte with which a specific binding agent will combine specifically
(b) exposing the immobilised target analyte to a mixture of the whole blood to be assayed and the specific binding agent with which said analyte combines specifically
(c) exposing the immobilised target analyte from step (b) to a secondary binding agent capable of binding to the specifically combined specific binding agent and to which colloidal gold particles have been bound
(d) exposing the immobilised target analyte from step (c) to a solution comprising silver ions whereby silver nucleates onto at least a portion of the gold bound to any bound secondary binding agent and
(e) relating the presence and/or amount of nucleated silver to the presence and/or amount of target analyte in the whole blood.

5. A method according to any one of Claim 1, 2, 3 or 4 wherein the target analyte comprises an antibody, protein, virus, hormone, drug, nucleic acid or polysaccharide.

6. A method according to Claim 1 wherein the target analyte is an immunoglobulin specific for HTLV-I, HIV, Hepatitis, Measles, Chicken Pox, Leukaemia or Rubella.

7. A method according to any one of the preceeding claims wherein the target analyte is a specific antibody and the immobilised binding agent is an antigen for which the said antibody is specific.

8. A method according to any of the Claims 1, 4, or 5 wherein the target analyte is a drug and the specific binding reagent used is an antibody specific for it.

9. A method according to any one of the above claims wherein the immobilised specific binding agent or immobilised target analyte comprises a microtitre plate, a microwell strip or a porous membrane coated with said agent or analyte.

10. A method according to any one of the preceeding claims wherein the gold labelled binding agent is an anti-human, anti-rat, anti-mouse, anti-guinea pig or anti-rabbit antibody directed to IgA, IgG, IgD or IgM immunoglobulins.

11. A method according to any of the above claims wherein the silver ion containing solution comprises silver lactate, silver citrate, silver nitrate or silver chloride reagents.

12. A method according to Claim 11 wherein the concentration of the silver ions in the solution is from 0.1 to 1000 mM.

13. A method according to Claim 12 wherein the concentration of the silver ions in the solution is from 1 to 100 mM.

14. A method according to Claim 13 wherein the concentration of the silver ions in the solution is from 2 to 20 mM.

15. A method according to Claim 11 wherein the silver ion containing solution also comprises a stabiliser and/or a retardant.

16. A method according to Claim 15 wherein the stabiliser comprises a reducing agent.

17. A method according to Claim 16 wherein the reducing agent is a hydroquinone.

18. A method according to Claim 15 wnerein the retardant comprises gum acacia.

19. A method according to Claim 17 wherein the hydroquinone is used at a concentration between 1% and 0.01% by weight.

20. A method according to Claim 18 wherein the gum acasia is used at a concentration of between 1% and 0.01% by weight.

21. A method according to any previous claim wherein the colloidal gold is 10nm or less in particle size.

22. A method according to Claim 21 wherein the colloidal gold is 5nm in particle size.

23. A method according to any one of Claim 1, 2, 3 or 4 wherein the gold labelled binding agent is incubated with the immobilised combined unlabelled agents for a period of from 10 to 60 minutes.

24. A method according to Claim 23 wherein the gold labelled binding agent is incubated with the immobilised combined unlabelled agents for a period of from 20 to 40 minutes.

25. A method according to Claim 24 wherein the gold labelled binding agent is incubated with the immobilised combined unlabelled agents for about 30 minutes.

26. A method according to one of Claims 1, 2, 3 or 4 wherein the silver solution is incubated with the completed immunogold assay for between 10 and 60 minutes.

27. A method according to Claim 26 wherein the silver solution is incubated with the completed immunogold assay for between 20 and 30 minutes.

28. A method according to Claim 1, 2, 3 or 4 wherein the immobilised agents are washed between each exposure.

29. A method according to Claim 29 wherein the washing is carried out using phosphate buffer saline or demineralised water.

30. A method according to Claim 1, 2, 3 or 4 wherein the amount of target analyte is determined by reading the absorbance at a specific wavelength of the completed silver enhanced immunogold assay surface and then relating that to the absorbance for a standard amount of analyte.

31. A method according to any one of the Claims 1, 2 or 5 wherein the presence of a silver deposit indicates the presence of the target analyte in the whole blood.

32. A method according to any one of the Claims 3 or Claim 4 or Claim 5 wherein the absence of a silver deposit indicates the presence of the target analyte in the whole blood.

33. A method according to any one of the Claims 1 to 30, 42 or 43 wherein the target analyte comprises an antibody to HIV or rubella or comprises theophylline or human growth hormone.

34. A kit for assaying analytes in whole blood according to the method of claim 2 comprising:
(a) specific binding agent with which a target analyte will combine specifically immobilised onto a solid substrate
(b) a secondary binding agent capable of binding to the combined target analyte and to which colloidal gold particles have been bound and
(c) a silver containing enhancer solution which causes silver to nucleate out onto gold particles.

35. A kit for assaying analytes in whole blood according to the method of claim 3 comprising:
(a) a specific binding agent with which a target analyte will combine specifically immobilised onto a solid substrate
(b) target analyte, or a specifically binding analogue thereof, to which colloidal gold particles have been bound and
(c) a silver containing enhancer solution which causes silver to nucleate out onto gold particles.

36. A kit for assaying analytes in whole blood according to the method of claim 4 comprising:
(a) a target analyte with which a specific binding agent will combine specifically immobilised onto a solid substrate
(b) said specific binding agent
(c) a secondary binding agent to which colloidal gold particles have been bound and which is capable of binding to the specific binding agent when that is bound to the immobilised target analyte and
(d) a silver containing enhancer solution which causes silver to nucleate out onto gold particles.

37. A kit according to any one of Claims 34, 35 or 36 further comprising a buffer solution and a solution of demineralised water.

38. A kit according to any one of the Claims 34 to 37 wherein the silver ion comprising solution comprises a soluble salt of silver.

39. A kit according to Claim 38 wherein the silver ion comprises a soluble organic silver salt.

40. A kit according to any one of Claims 34 to 39 wherein there is further included a stabiliser and/or a retardant for the silver ion comprising solution.

41. A kit according to Claim 40 wherein said stabiliser comprises a reducing reagent.

42. A kit according to Claim 41 wherein said reducing agent comprises a hydroquinone.

43. A kit according to Claim 40 wherein the retardant comprises gum acacia.

44. A kit according to any one of the Claims 34 to 43 wherein the silver ion comprising solution comprises silver lactate, silver citrate, silver nitrate or silver chloride.

45. A kit according to any one of the Claims 34 to 44 wherein specific binding agent for an antibody to HIV or rubella or specific binding agent for theophylline or human growth hormone is included.

## Patentansprüche

1. Verfahren, um Immunogoldassays, die spezifisch bindende, auf festen Substraten immobilisierte Agentien verwenden und die mit Vollblut durchgeführt werden, bei visueller und/oder photometrischer und/oder colorimetrischer Auswertung in ihrer Färbung zu verstärken, das folgende Schritte umfaßt:
Inkontaktbringen der Endproben aus dem Immunogoldassay mit einer Silberionen enthaltenden Lösung, wobei Silber auf mindestens einem Teil des Goldes abgeschieden wird, und
anschließend Korrelieren des Vorhandenseins und/oder der Menge des abgeschiedenen Silbers mit dem Vorhandensein und/oder der Menge der zu analysierenden Substanz.

2. Verfahren zur qualitativen oder quantitativen Bestimmung einer zu analysierenden Substanz in Vollblut, das folgende Schritte umfaßt:
(a) Immobilisierung eines spezifisch bindenden Agens, mit dem die zu analysierende Substanz durch eine spezifische Bindung kombiniert, auf einem festen Substrat,
(b) Inkontaktbringen des immobilisierten spezifisch bindenden Agens mit einer zu untersuchenden Vollblutprobe,
(c) Inkontaktbringen des immobilisierten spezifisch bindenden Agens aus Schritt (b) mit einem zweiten bindenden Agens, das eine Bindung mit der spezifisch kombinierten zu analysierenden Substanz eingehen kann und an das kolloidale Goldteilchen gebunden wurden,
(d) Inkontaktbringen des immobilisierten spezifisch bindenden Agens aus Schritt (c) zu einer Silberionen enthaltenden Lösung, wobei sich das Silber auf mindestens einem Teil des Goldes abscheidet, das an einen gebundenen zweiten bindenden Reagens gebunden ist, und
(e) Korrelation des Vorhandenseins und/oder der Menge des abgeschiedenen Silbers mit dem Vorhandensein und/oder der Menge der zu analysierenden Substanz im Vollblut.

3. Verfahren zur qualitativen und/oder quantitativen Bestimmung einer zu analysierenden Substanz in Vollblut, das folgende Schritte umfaßt:
(a) Immobilisierung eines spezifisch bindenden Agens, mit dem die zu analysierende Substanz durch eine spezifische Bindung kombiniert, auf einem festen Substrat,
(b) Inkontaktbringen des spezifisich bindenden Agens mit einem Gemisch aus Vollblut und einer Lösung, die einen Anteil der zu analysierenden Substanz oder eine ihr analoge Substanz, die spezifisch an das spezifisch bindende Agens binden und an die kolloidale Goldteilchen gebunden wurden, enthält,
(c) Inkontaktbringen des immobilisierten spezifisch bindenden Agens aus Schritt (b) mit einer Silberionen enthaltenden Lösung, wobei sich das Silber auf mindestens einen Teil des Goldes abscheidet, das über eine markierte zu analysierende Substanz oder eine ihr analoge Substanz an das bindende Agens gebunden ist, und
(d) Korrelation des Vorhandenseins und/oder der Menge von abgeschiedenem Silber mit dem Vorhandensein und/oder der Menge der zu analysierenden Substanz im Vollblut.

4. Verfahren zur qualitativen und/oder quantitativen Bestimmung einer zu analysierenden Substanz in Vollblut, das folgende Schritte umfaßt:
(a) Immobilisierung einer zu analysierenden Substanz, mit der ein spezifisch bindendes Agens über eine spezifische Bindung kombiniert, auf einem festen Substrat,
(b) Inkontaktbringen der immobilisierten zu analysierenden Substanz zu einem Gemisch aus dem zu analysierenden Vollblut und dem spezifisch bindenden Agens, mit dem die Substanz durch eine spezifische Bindung kombiniert,
(c) Inkontaktbringen der immobilisierten zu analysierenden Substanz aus Schritt (b) mit einem zweiten bindenden Agens, das an das spezifisch kombinierte spezifisch bindende Agens gebunden werden kann und an das kolloidale Goldteilchen gebunden worden sind,
(d) Inkontaktbringen der immobilisierten zu analysierenden Substanz aus Schritt (c) mit einer Silberionen enthaltenden Lösung, wobei Silber sich auf mindestens einem Teil des Goldes abscheidet, das an ein gebundenes zweites bindendes Agens gebunden ist, und
(e) Korrelation des Vorhandenseins und/oder der Menge Anteils des abgeschiedenen Silbers mit dem Vorhandensein und/oder der Menge der zu analysierenden Substanz im Vollblut.

5. Verfahren nach einem der Ansprüche 1 bis 4, wobei die zu analysierende Substanz ein Antikörper, ein Protein, ein Virus, ein Hormon, ein Arzneimittel, eine Nucleinsäure oder ein Polysaccharid ist.

6. Verfahren nach Anspruch 1, wobei die zu analysierende Substanz ein für HTLV-I, HIV, Hepatitis, Masern, Windpocken, Leukämie oder Rubella spezifisches Immunglobulin ist.

7. Verfahren nach einem der vorhergehenden Ansprüche, wobei die zu analysierende Substanz ein spezifischer Antikörper ist und das immobilisierte bindende Agens ein Antigen ist, für das der Antikörper spezifisch ist.

8. Verfahren nach Anspruch 1, 4 oder 5, wobei die zu analysierende Substanz ein Arzneimittel ist und das verwendete spezifisch bindende Agens ein dafür spezifischer Antikörper ist.

9. Verfahren nach einem der vorhergehenden Ansprüche, wobei das immobilisierte spezifisch bindende Agens oder die immobilisierte zu analysierende Substanz in Form einer Beschichtung auf einer Mikrotiterplatte, einem Streifen mit Mikrovertiefungen oder einer porösen Membran vorliegen.

10. Verfahren nach einem der vorhergehenden Ansprüche, wobei das mit Gold markierte bindende Agens ein Antikörper ist, der gegen IgA, IgG, IgD oder IgM-Immunglobuline des Menschen, der Ratte, der Maus, des Meerschweinchens oder des Kaninchens gerichtet ist.

11. Verfahren nach einem der vorhergehenden Ansprüche, wobei die Silberionen enthaltende Lösung Silberlactat, Silbercitrat, Silbernitrat oder Silberchlorid als Reagentien enthält.

12. Verfahren nach Anspruche 11, wobei die Silberionenkonzentration in der Lösung in einem Bereich von 0,1 bis 1000 mM liegt.

13. Verfahren nach Anspruch 12, wobei die Silberionenkonzentration in der Lösung in einem Bereich von 1 bis 100 mM liegt.

14. Verfahren nach Anspruch 13, wobei die Silberionenkonzentration in der Lösung in einem Bereich von 2 bis 20 mM liegt.

15. Verfahren nach Anspruch 11, wobei die Silberionen enthaltende Lösung ferner ein Stabilisierungsmittel und/oder einen Verzögerer enthält.

16. Verfahren nach Anspruch 15, wobei das Stabilisierungsmittel ein Reduktionsmittel ist.

17. Verfahren nach Anspruch 16, wobei das Reduktionsmittel ein Hydrochinon ist.

18. Verfahren nach Anspruch 15, wobei der Verzögerer Gummi arabicum ist.

19. Verfahren nach Anspruch 17, wobei das Hydrochinon in einer Konzentration von 1 bis 0,01 Gew.-% eingesetzt wird.

20. Verfahren nach Anspruch 18, wobei das Gummi arabicum in einer Konzentration von 1 bis 0,01 Gew.-% eingesetzt wird.

21. Verfahren nach einem der vorhergehenden Ansprüche, wobei das kolloidale Gold eine Teilchengröße von 10 nm oder weniger aufweist.

22. Verfahren nach Anspruch 21, wobei das kolloidale Gold eine Teilchengröße von 5 nm aufweist.

23. Verfahren nach einem der Ansprüche 1 bis 4, wobei das mit Gold markierte bindende Agens mit den immobilisierten kombinierten nichtmarkierten Agentien 10 bis 60 min inkubiert wird.

24. Verfahren nach Anspruch 23, wobei das mit Gold markierte bindende Agens mit den immobilisierten kombinierten nichtmarkierten Agentien für einen Zeitraum von 20 bis 40 min inkubiert wird.

25. Verfahren nach Anspruch 24, wobei das mit Gold markierte bindende Agens mit den immobilisierten kombinierten nichtmarkierten Agentien etwa 30 min inkubiert wird.

26. Verfahren nach einem der Ansprüche 1 bis 4, wobei die Silberlösung mit dem fertig durchgeführten Immunogoldassay 10 bis 60 min inkubiert wird.

27. Verfahren nach Anspruch 26, wobei die Silberlösung mit dem fertig durchgeführten Immunogoldassay 20 bis 30 min inkubiert wird.

28. Verfahren nach einem der Ansprüche 1 bis 4, wobei die immobilisierten Agentien zwischen jedem Inkontaktbringen gewaschen werden.

29. Verfahren nach Anspruch 28, wobei das Waschen unter Verwendung von phosphatgepufferter Kochsalzlösung oder entmineralisiertem Wasser durchgeführt wird.

30. Verfahren nach einem der Ansprüche 1, 2, 3 oder 4, wobei die Menge der zu analysierender Substanz durch Ermittlung der Absorption der Oberfläche des mit Silber verstärkten fertig durchgeführten Immunogoldassays bei einer bestimmten Wellenlänge und durch anschließende Korrelation mit der Absorption einer standardisierten Menge der zu analysierenden Substanz bestimmt wird.

31. Verfahren nach einem der Ansprüche 1, 2 und 5, wobei das Vorhandensein einer Silberabscheidung das Vorhandensein der zu analysierenden Substanz im Vollblut anzeigt.

32. Verfahren nach einem der Ansprüche 3, 4 und 5, wobei die Abwesenheit einer Silberabscheidung das Vorhandensein der zu analysierenden Substanz im Vollblut anzeigt.

33. Verfahren nach einem der Ansprüche 1 bis 33, wobei die zu analysierende Substanz ein Antikörper gegen HIV oder Rubella, Theophyllin oder menschliches Wachstumshormon ist.

34. Kit zur Bestimmung von zu analysierenden Substanzen in Vollblut nach dem Verfahren von Anspruch 2, der enthält:
(a) ein auf einem festen Substrat immobilisiertes spezifisch bindendes Agens, mit dem die zu analysierende Substanz über eine spezifische Bindung kombiniert,
(b) ein zweites bindendes Agens, das eine Bindung mit der kombinierten zu analysierenden Substanz eingehen kann und an das kolloidale Goldteilchen gebunden wurden, und
(c) eine Silber enthaltende Verstärkerlösung, aus der sich Silber auf Goldteilchen abscheidet.

35. Kit zur Bestimmung von zu analysierenden Substanzen in Vollblut nach dem Verfahren von Anspruch 3, der enthält:
(a) ein auf einem festen Substrat immobilisiertes spezifisch bindendes Agens, mit dem die zu analysierende Substanz über eine spezifische Bindung kombiniert,
(b) eine zu analysierende Substanz oder eine zu ihr analoge spezifisch bindende Substanz, an die kolloidale Goldteilchen gebunden wurden, und
(c) eine Silber enthaltende Verstärkerlösung, aus der sich Silber auf Goldteilchen abscheidet.

36. Kit zur Bestimmung von zu analysierenden Substanzen in Vollblut nach dem Verfahren von Anspruch 4, der enthält:
(a) eine auf einem festen Substrat immobilisierte zu analysierende Substanz, mit der ein spezifisch bindendes Agens über eine spezifische Bindung kombiniert,
(b) das spezifisch bindende Agens,
(c) ein zweites bindendes Agens, an das kolloidale Goldteilchen gebunden wurden und das sich an das spezifisch bindende Agens binden kann, wenn dieses an die immobilisierte zu analysierende Substanz gebunden ist, und
(d) eine Silber enthaltenden Verstärkerlösung, aus der sich Silber auf Goldteilchen abscheidet.

37. Kit nach einem der Ansprüche 34 bis 36, der ferner eine Pufferlösung und eine Lösung aus entmineralisiertem Wasser enthält.

38. Kit nach einem der Ansprüche 34 bis 37, wobei die Silberionen enthaltende Lösung ein lösliches Silbersalz enthält.

39. Kit nach Anspruch 38, wobei die Silberionen enthaltende Lösung ein lösliches organisches Silbersalz enthält.

40. Kit nach einem der Ansprüche 34 bis 39, wobei ferner ein Stabilisierungsmittel und/oder ein Verzögerer für die Silberionen enthaltende Lösung enthalten sind.

41. Kit nach Anspruch 40, wobei das Stabilisierungsmittel ein Reduktionsmittel ist.

42. Kit nach Anspruch 41, wobei das Reduktionsmittel ein Hydrochinon ist.

43. Kit nach Anspruch 40, wobei der Verzögerer Gummi arabicum umfaßt.

44. Kit nach einem der Ansprüche 34 bis 43, wobei die Silberionen enthaltende Lösung Silberlactat, Silbercitrat, Silbernitrat oder Silberchlorid enthält.

45. Kit nach einem der Ansprüche 34 bis 44, der ein spezifisch bindendes Agens für einen HIV- oder Rubella-Antikörper oder ein spezifisch bindendes Agens für Theophyllin oder menschliches Wachstumshormon enthält.

## Revendications

1. Procédé pour amplifier visuellement et/ou photométriquement et/ou colorimétriquement des essais de dosage de type "immuno-or", qui utilisent des agents spécifiques de liaison immobilisés sur des substrats solides et qui sont effectués sur du sang entier, ce procédé comprenant l'exposition des tests d'essais de dosage de type "immuno-or" à une solution comprenant des ions argent, de sorte que l'argent se fixe sur au moins une partie de l'or, et l'on établit ensuite une corrélation entre la présence et/ou la quantité d'argent fixée, d'une part, et la présence et/ou la quantité de l'analyte constituant une cible, d'autre part.

2. Procédé pour la détermination de la présence ou de la quantité d'un analyte constituant une cible dans du sang entier, le procédé comprenant :
(a) l'immobilisation sur un substrat solide d'un agent de liaison spécifique avec lequel l'analyte constituant une cible va se combiner spécifiquement,
(b) l'exposition de l'agent de liaison spécifique, immobilisé, à un échantillon de sang entier à doser ou à examiner,
(c) l'exposition de l'agent de liaison spécifique, immobilisé, provenant de l'étape (b), à un agent de liaison secondaire capable de se fixer sur l'analyte constituant une cible, combiné spécifiquement, et auquel des particules d'or colloïdal ont été liées ou fixées,
(d) l'exposition de l'agent de liaison spécifique immobilisé, provenant de l'étape (c), à une solution contenant des ions argent, de sorte que les ions argent se fixent et se regroupent sur au moins une partie de l'or lié sur n'importe quel réactif de liaison secondaire lié, et
(e) l'établissement d'une corrélation entre la présence et/ou la quantité d'argent fixée par agglomération ou nucléation avec la présence et/ou la quantité de l'analyte constituant une cible dans le sang entier.

3. Procédé pour la détermination de la présence et/ou de la quantité d'un analyte constituant une cible dans du sang entier, ce procédé comprenant :
(a) l'immobilisation sur un substrat solide d'un agent de liaison spécifique avec lequel l'analyte constituant une cible va se combiner spécifiquement,
(b) l'exposition de l'agent de liaison spécifique à un mélange de sang entier avec une solution contenant une certaine quantité de l'analyte constituant une cible, ou d'un de ses analogues, qui va se lier spécifiquement audit agent de liaison spécifique auquel des particules d'or colloïdal ont été fixées ou liées,
(c) l'exposition de l'agent de liaison spécifique, immobilisé, provenant de l'étape (b) à une solution comprenant des ions argent, de sorte que l'argent se fixe par agglomération sur au moins une partie de n'importe quel or fixé sur ledit agent de liaison, par l'intermédiaire de n'importe quel analyte constituant une cible ou de son analogue, marqué, et
(d) l'établissement d'une corrélation entre la présence et/ou la quantité d'argent fixée par agglomération ou nucléation et la présence et/ou la quantité de l'analyte constituant une cible dans le sang entier.

4. Procédé pour la détermination de la présence et/ou de la quantité d'un analyte constituant une cible dans du sang entier, ce procédé comprenant :
(a) l'immobilisation sur un substrat solide d'un analyte constituant une cible avec lequel un agent de liaison spécifique va se combiner spécifiquement,
(b) l'exposition de l'analyte constituant une cible, immobilisé, à un mélange du sang entier à examiner et de l'agent de liaison spécifique avec lequel ledit analyte se combine spécifiquement,
(c) l'exposition de l'analyte constituant une cible, immobilisé, provenant de l'étape (b), à un agent de liaison secondaire capable de se fixer sur l'agent de liaison spécifique spécifiquement combiné et auquel des particules d'or colloïdal ont été fixées,
(d) l'exposition de l'analyte constituant une cible, immobilisé, provenant de l'étape (c), à une solution comprenant des ions argent, de sorte que l'argent se fixe par agglomération sur au moins une partie de l'or lié à tout agent de liaison secondaire lié, et
(e) l'établissement d'une corrélation entre la présence et/ou la quantité de l'argent fixé par agglomération ou nucléation avec la présence et/ou la quantité de l'analyte constituant une cible dans le sang entier.

5. Procédé selon l'une quelconque des revendications 1, 2, 3 ou 4, dans lequel l'analyte constituant une cible comprend un anticorps, de la protéine, du virus, une hormone, un médicament, de l'acide nucléique ou un polysaccharide.

6. Procédé selon la revendication 1, dans lequel l'analyte constituant une cible est une immunoglobuline spécifique pour HTLV-1, HIV (Sida), de l'hépatite, de la rougeole, de la varicelle, de la leucémie ou de la rubéole.

7. Procédé selon l'une quelconque des revendications précédentes, dans lequel l'analyte constituant une cible est un anticorps spécifique, et l'agent de liaison, immobilisé, est un antigène pour lequel ledit anticorps est spécifique.

8. Procédé selon l'une quelconque des revendications 1, 4 ou 5, dans lequel l'analyte constituant une cible est un médicament, et le réactif à rôle de liaison spécifique que l'on utilise est un anticorps spécifique du médicament.

9. Procédé selon l'une quelconque des revendications précédentes, dans lequel l'agent de liaison spécifique, immobilisé, ou l'analyte servant de cible, immobilisé, comprend une plaque pour microtitrage, une bande comportant des microcreux ou une membrane poreuse revêtue dudit agent ou dudit analyte.

10. Procédé selon l'une quelconque des revendications précédentes, dans lequel l'agent de liaison marqué par de l'or est un anticorps anti-humain, anti-rat, anti-souris, anti-cobaye ou anti-lapin, dirigé vers des immunoglobulines IgA, IgG, IgD ou IgM.

11. Procédé selon l'une quelconque des revendications ci-dessus, dans lequel la solution contenant de l'ion argent comprend des réactifs qui sont le lactate d'argent, le citrate d'argent, le nitrate d'argent ou le chlorure d'argent.

12. Procédé selon la revendication 11, dans lequel la concentration des ions argent dans la solution est de 0,1 à 1 000 mM.

13. Procédé selon la revendication 12, dans lequel la concentration des ions argent dans la solution est de 1 à 100 mM.

14. Procédé selon la revendication 13, dans lequel la concentration des ions argent dans la solution est de 2 à 20 mM.

15. Procédé selon la revendication 11, dans lequel la solution contenant de l'ion argent comprend aussi un stabilisant et/ou un retardateur.

16. Procédé selon la revendication 15, dans lequel le stabilisant est constitué par ou comprend un agent réducteur.

17. Procédé selon la revendication 16, dans lequel l'agent réducteur est une hydroquinone.

18. Procédé selon la revendication 15, dans lequel le retardateur est constitué par ou comprend de la gomme arabique.

19. Procédé selon la revendication 17, dans lequel l'hydroquinone est utilisée en une concentration comprise entre 1 % et 0,01 % en poids.

20. Procédé selon la revendication 18, dans lequel on utilise de la gomme arabique à une concentration comprise entre 1 % et 0,01 % en poids.

21. Procédé selon l'une quelconque des revendications précédentes, dans lequel l'or colloïdal a une taille particulaire égale ou inférieure à 10 nm.

22. Procédé selon la revendication 21, dans lequel l'or colloïdal a une taille particulaire de 5 nm.

23. Procédé selon l'une quelconque des revendications 1, 2, 3 ou 4, dans lequel l'agent de liaison marqué par de l'or est soumis à incubation pendant une période de 10 à 60min avec les agents non marqués, combinés et immobilisés.

24. Procédé selon la revendication 23, dans lequel l'agent de liaison marqué par de l'or est soumis à incubation pendant une période de 20 à 40 min avec les agents non marqués, combinés et immobilisés.

25. Procédé selon la revendication 24, dans lequel l'agent de liaison ou de fixation marqué par de l'or est mis à incuber durant 30 min environ avec les agents non marqués, combinés et immobilisés.

26. Procédé selon l'une quelconque des revendications 1, 2 ou 3 ou 4, dans lequel la solution d'argent est mise à incuber pendant une période comprise entre 10 et 60 min avec le milieu de dosage de type "immuno-or" achevé.

27. Procédé selon la revendication 26, dans lequel la solution d'argent est mise à incuber pendant une période comprise entre 20 et 30 min avec le milieu de l'essai de dosage de type "immuno-or" achevé.

28. Procédé selon la revendication 1, 2, 3 ou 4, dans lequel les agents immobilisés sont lavés entre chaque exposition.

29. Procédé selon la revendication 28, dans lequel le lavage est effectué à l'aide d'un tampon de phosphate, d'une solution saline ou d'eau déminéralisée.

30. Procédé selon la revendication 1, 2, 3 ou 4, dans lequel la quantité de l'analyte constituant une cible est déterminée par lecture de l'absorptance à une longueur d'onde spécifique de la surface pour dosage de type immuno-or achevé, avec amplification par de l'argent, puis l'on effectue une corrélation avec l'absorptance d'une quantité normalisée de l'analyte.

31. Procédé selon l'une quelconque des revendications 1, 2 ou 5, dans lequel la présence d'un dépôt d'argent indique la présence de l'analyte constituant une cible dans le sang entier.

32. Procédé selon l'une quelconque des revendications 3 ou 4 ou 5, dans lequel l'absence d'un dépôt d'argent indique la présence de l'analyte constituant une cible dans le sang entier.

33. Procédé selon l'une quelconque des revendications 1 à 32, dans lequel l'analyte constituant une cible comprend un anticorps de HIV (Sida) ou de la rubéole ou comprend de la théophylline ou de l'hormone de croissance humaine.

34. Nécessaire pour la détection et le dosage d'analytes dans du sang entier selon le procédé de la revendication 2, ce nécessaire comprenant :
(a) un agent de liaison spécifique avec lequel un analyte constituant une cible va se combiner spécifiquement en étant immobilisé sur un substrat solide,
(b) un agent de liaison secondaire, capable de se fixer sur l'analyte combiné constituant une cible et sur lequel des particules d'or colloïdal ont été fixées, et
(c) une solution d'amplificateur contenant de l'argent, qui provoque le dépôt de fixation d'argent sur les particules d'or.

35. Nécessaire pour déceler et doser des analytes dans du sang entier, selon le procédé de la revendication 3, ce nécessaire comprenant :
(a) un agent de liaison spécifique avec lequel un analyte constituant une cible va se combiner en étant spécifiquement immobilisé sur un substrat solide,
(b) de l'analyte constituant une cible ou son analogue à liaison spécifique, sur lequel des particules d'or colloïdal ont été fixées, et
(c) une solution amplificatrice contenant de l'argent, qui provoque le dépôt de fixation de l'argent sur des particules d'or.

36. Nécessaire pour déceler ou doser des analytes dans du sang entier selon le procédé de la revendication 4, ce nécessaire comprenant :
(a) un analyte constituant une cible avec lequel un agent de liaison spécifique va se combiner en étant spécifiquement immobilisé sur un substrat solide,
(b) ledit agent de liaison spécifique,
(c) un agent de liaison secondaire sur lequel des particules d'or colloïdal ont été fixées et qui est capable de se lier ou se finir à l'agent spécifique de liaison quand il est lié à l'analyte immobilisé constituant une cible, et
(d) une solution amplificatrice contenant de l'argent, qui provoque le dépôt de fixation de l'argent sur les particules d'or.

37. Nécessaire selon l'une quelconque des revendications 34, 35 ou 36, comprenant en outre une solution tampon et une solution d'eau déminéralisée.

38. Nécessaire selon l'une quelconque des revendications 34 à 37, dans lequel la solution comprenant de l'ion argent comprend un sel soluble de l'argent.

39. Nécessaire selon la revendication 38, dans lequel le composé contenant l'ion argent comprend un sel organique soluble de l'argent

40. Nécessaire selon l'une quelconque des revendications 34 à 39, dans lequel est également inclus un stabilisant et/ou un retardateur pour la solution contenant de l'ion argent.

41. Nécessaire selon la revendication 40, dans lequel ledit stabilisant comprend un réactif réducteur.

42. Nécessaire selon la revendication 41, dans lequel ledit agent réducteur comprend une hydroquinone.

43. Nécessaire selon la revendication 40, dans lequel le retardateur comprend de la gomme arabique.

44. Nécessaire selon l'une quelconque des revendications 34 à 43, dans lequel la solution comprenant de l'ion argent comprend du lactate d'argent, du citrate d'argent, du nitrate d'argent ou du chlorure d'argent.

45. Nécessaire selon l'une quelconque des revendications 34 à 44, dans lequel est inclus un agent de liaison spécifique pour un anticorps de HIV (Sida) ou de la rubéole ou un agent de liaison spécifique pour la théophylline ou pour l'hormone de croissance humaine.
